(19) Europäisches Patentamt
European Patent Office
Office européen des brevets

(11) Publication number: **0 107 890**
**B1**

(12) # EUROPEAN PATENT SPECIFICATION

(45) Date of publication of patent specification: **29.01.86**

(51) Int. Cl.[4]: **C 07 K 7/10**, C 07 K 7/26, A 61 K 37/02, A 23 K 1/16

(21) Application number: **83303490.3**

(22) Date of filing: **16.06.83**

(54) **Mammalian PGRF.**

(30) Priority: **16.06.82 PCt/us82/00812**
**15.09.82 US 418248**

(43) Date of publication of application:
**09.05.84 Bulletin 84/19**

(45) Publication of the grant of the patent:
**29.01.86 Bulletin 86/05**

(84) Designated Contracting States:
**AT BE CH DE FR GB IT LI LU NL SE**

(56) References cited:

**CHEMICAL ABSTRACTS, vol. 98, 1983, page 103, abstract no. 101522a, COLUMBUS OHIO (US)**
**CHEMICAL ABSTRACTS, vol. 98, 1983, page 102, abstract no. 101520y, COLUMBUS OHIO (US)**
**CHEMICAL ABSTRACTS, vol. 99, 1983, page 111, abstract no. 64725s, COLUMBUS OHIO (US)**

(73) Proprietor: **THE SALK INSTITUTE FOR BIOLOGICAL STUDIES**
**10010 North Torrey Pines Road**
**La Jolla California 92038 (US)**

(72) Inventor: **Ling, Nicholas Chai-Kwan**
**5324 Bloch Street**
**San Diego California 92122 (US)**
Inventor: **Esch, Frederick Stephen**
**2929 Fire Mountain Drive No. 69**
**Oceanside California 92054 (US)**
Inventor: **Bohlen, Peter**
**147 Honeycomb Court**
**Encinitas California 92024 (US)**
Inventor: **Brazeau, Paul Ernest, Jr.**
**7758 Camino Noguera**
**San Diego California 92122 (US)**
Inventor: **Guillemin, Roger Charles Louis**
**7316 Encelia Drive**
**La Jolla California 92037 (US)**

(74) Representative: **Lawrence, Malcolm Graham et al**
**BROOKES & MARTIN High Holborn House 52/54 High Holborn**
**London WC1V 6SE (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

CHEMICAL ABSTRACTS, vol. 99, 1983, page 543, abstract no. 69632u, COLUMBUS OHIO (US)
UNLISTED DRUGS, vol. 35, no. 3, March 1983, (US), Spec.Libr.Assoc., hpGRF

**Description**

The present invention relates to a peptide having influence on the function of the pituitary gland in humans and other mammals. In particular, the present invention is directed to a peptide which promotes the release of growth hormone by the pituitary gland.

Background of the invention

Since the early 1950's physiologists and clinicians have recognized that the hypothalamus of the brain controls all the secretory functions of the adenohypophysis. This control is neurohumoral, with specialized neurosecretory neurons in the hypothalamus producing special polypeptides, the effect and role of each of which is to trigger acutely and chronically the secretion of each pituitary hormone. To this day, a hypothalamic releasing factor has been characterized for the pituitary hormones thyrotropin and prolactin (the tripeptide TRF), for the pituitary gonadotropins luteinizing hormone and follicle stimulating hormone (the decapeptide LRF, LH-RH, GnRH or Gn-RF) and for the pituitary hormones β-endorphin and adrenocorticotropin (the 41-amino acid polypeptide CRF). In addition, an inhibitory factor has been characterized: hypothalamic somatostatin inhibits, at the pituitary level, the secretion of growth hormone. Each of these hypothalamic releasing factors and somatostatin have been reproduced by total synthesis, and many analogs of the native structures have been synthesized, some with far greater activity than the natural compounds.

To this day, a corresponding hypothalamic releasing factor for the pituitary growth hormone or somatotropin has not been characterized, even though there has been extensive physiological and clinical evidence for its existence. One of the major problems in the isolation and characterization of the hypothalamic growth hormone releasing factor (hereinafter GRF) is that the active peptide appears to be present in each hypothalamic fragment in infinitesimal amounts which we believe to be of the order of 50—150 femtomoles. This is far less than anything ever calculated for the other hypothalamic releasing factors. In keeping with this statement is the corollary that hypothalamic GRF is of extremely high potency.

Another major problem in the isolation of hypothalamic GRF has been the presence in hypothalamic extracts of very large amounts of somatostatin which of course prevent or would give aberrant results in any attempted bioassay. Over the last few years, several laboratories have claimed to have isolated and characterized the hypothalamic GRF; all these claims were dealing with artifacts as recognized later by the authors (Schally, A. V. S. et al. *J. Biol. Chem.* 246, 6647, 1971; Veber D. F. et al., *Biochem. Biophys. Res. Commun.* 45, 235, 1971). Such incorrect claims can be explained in part, by the difficulty of the bioassays involved in assessing release of growth hormone.

Summary of the invention

A 44-residue polypeptide has been isolated from a human islet cell tumor, purified, characterized, synthesized and tested which promotes the release of growth hormone(GH) by the pituitary. This peptide has the sequence:

H-Tyr-Ala-Asp-Ala-Ile-Phe-Thr-Asn-Ser-Tyr-Arg-Lys-Val-Leu-
Gly-Gln-Leu-Ser-Ala-Arg-Lys-Leu-Leu-Gln-Asp-Ile-Met-Ser-
Arg-Gln-Gln-Gly-Glu-Ser-Asn-Gln-Glu-Arg-Gly-Ala-Arg-Ala-Arg-Leu-$NH_2$.

It is believed to be and is hereinafter referred to as PGRF (for human pancreatic GRF) and will also be termed somatocrinin. Two other highly purified peptides were also isolated along therewith which exhibit GH-releasing activity and which are PGRF(1—37) free acid and PGRF(1—40) free acid. These peptides can be used to promote the growth of warm-blooded animals and of cold-blooded animals in aquiculture.

Pharmaceutical compositions in accordance with the invention include PGRF, an analog or biologically active fragments thereof, or a nontoxic salt of any of the foregoing, dispersed in a pharmaceutically acceptable liquid or solid carrier. Such pharmaceutical compositions can be used in clinical medicine, both human and veterinary, in acute or chronic administration for diagnostic or therapeutic purposes.

Detailed description of preferred embodiments

The nomenclature used to define the peptides is that specified by Schroder & Lubke, "The Peptides", Academic Press (1965), wherein in accordance with conventional representation the amino group at the N-terminal appears to the left and the carboxyl group at the C-terminal to the right. Where the amino acid residue has isomeric forms, it is the L-form of the amino acid that is represented unless otherwise expressly indicated.

The invention provides synthetic PGRF peptides having the following formula:

H-Tyr-Ala-Asp-Ala-Ile-Phe-Thr-Asn-Ser-Tyr-Arg-Lys-Val-Leu-
Gly-Gln-Leu-Ser-Ala-Arg-Lys-Leu-Leu-Gln-Asp-Ile-Met-Ser-
Arg-Gln-Gln-Gly-Glu-$R_{34}$-Asn-Gln-Glu-$R_{38}$-Gly-$R_{40}$-$R_{41}$-R

wherein R is OH or $NH_2$, $R_{34}$ is Ser or Ala, $R_{38}$ is Arg or Ser, $R_{40}$ is Ala or Arg, and $R_{41}$ is Arg, Arg-Ala,

Arg-Ala-Arg, Arg-Ala-Arg-Leu or des-$R_{41}$. Also included are biologically active fragments where R can also be either OH or $NH_2$.

The peptides are synthesized by a suitable method, such as by exclusively solid-phase techniques, by partial solid-phase techniques, by fragment condensation, by classical solution couplings, or by the employment of recently developed recombinant DNA techniques. For example, the techniques of exclusively solid-phase synthesis are set forth in the textbook "Solid-Phase Peptide Synthesis", Stewart & Young, Freeman & Co., San Francisco, 1969, and are exemplified by the disclosure of U.S. Patent No. 4,105,603, issued August 8, 1978 to Vale et al. The fragment condensation method of synthesis is exemplified in U.S. Patent No. 3,972,859 (August 3, 1976). Other available syntheses are exemplified by U.S. Patent No. 3,842,067 (October 15, 1974) and U.S. Patent No. 3,862,925 (January 28, 1975). Production of the synthetic peptides using recombinant DNA techniques will likely be used to satisfy large-scale commercial requirements.

Common to coupling-type synthesis is the protection of the labile side chain groups of the various amino acid moieties with suitable protecting groups which will prevent a chemical reaction from occurring at that site until the group is ultimately removed. Usually also common is the protection of an alpha-amino group on an amino acid or a fragment while that entity reacts at the carboxyl group, followed by the selective removal of the alpha-amino protecting group to allow subsequent reaction to take place at that location. Accordingly, it is common that, as a step in the synthesis, an intermediate compound is produced which includes each of the amino acid residues located in its desired sequence in the peptide chain with side-chain protecting groups linked to the appropriate residues.

Also considered to be within the scope of the present invention are intermediates of the formula:

$X^1$-Tyr($X^2$)-Ala-Asp($X^3$)-Ala-Ile-Phe-Thr($X^4$)-Asn-Ser($X^5$)-Tyr($X^2$)-Arg($X^6$)-Lys($X^7$)-
Val-Leu-Gly-Gln-Leu-Ser($X^5$)-Ala-Arg($X^6$)-Lys($X^7$)-Leu-Leu-Gln-Asp($X^3$)-Ile-Met-Ser($X^5$)-
Arg($X^6$)-Gln-Gln-Gly-Glu($X^3$)-$R_{34}$($X^5$)-Asn-Gln-Glu($X^3$)-$R_{38}$($X^5$ or $X^6$)-Gly-$R_{40}$($X^6$)-
Arg($X^6$)-Ala-Arg($X^6$)-Leu-$X^8$

wherein: $X^1$ is either hydrogen or an α-amino protecting group. The α-amino protecting groups contemplated by $X^1$ are those known to be useful in the art of step-wise synthesis of polypeptides. Among the classes of α-amino protecting groups covered by $X^1$ are (1) acyl-type protecting groups, such as formyl, trifluoroacetyl, phthalyl, toluenesulfonyl(Tos), benzensulfonyl, nitrophenylsulfenyl, tritylsulfenyl, o-nitrophenoxyacetyl, chloroacetyl, acetyl, and γ-chlorobutyryl; (2) aromatic urethan-type protecting groups, such as benzyloxycarbonyl(Z) and substituted Z, such as p-chlorobenzyloxycarbonyl, p-nitrobenzyloxycarbonyl, p-bromobenzyloxycarbonyl, p-methoxybenzyloxycarbonyl; (3) aliphatic urethan protecting groups, such as t-butyloxycarbonyl (BOC), diisopropylmethyloxycarbonyl, isopropyloxycarbonyl, ethoxycarbonyl, allyloxycarbonyl; (4) cycloalkyl urethan-type protecting groups, such as cyclopentyloxycarbonyl, adamantyloxycarbonyl, and cyclohexyloxycarbonyl; (5) thiourethan-type protecting groups, such as phenylthiocarbonyl; (6) alkyl-type protecting groups, such as triphenylmethyl (trityl), benzyl; (7) trialkylsilane groups, such as trimethylsilane. The preferred α-amino protecting group is BOC.

$X^2$ is a protecting group for the phenolic hydroxyl group of Tyr selected from the group consisting of tetrahydropyranyl, tert-butyl, trityl, Bzl, CBZ, 4Br-CBZ and 2,6-dichlorobenzyl. The preferred protecting group is 2,6-dichlorobenzyl. $X^2$ can be hydrogen which means that there is no protecting group on the hydroxyl group.

$X^3$ is hydrogen or an ester-forming protecting group for the carboxyl group of Asp or Glu and is selected from the group consisting of Bzl, 2,6-dichlorobenzyl, methyl and ethyl.

$X^4$ and $X^5$ are protecting groups for the hydroxyl group of Thr and Ser and are selected from the group consisting of acetyl, benzoyl, tert-butyl, trityl, tetrahydropyranyl, Bzl, 2,6-dichlorobenzyl and CBZ. The preferred protecting group is Bzl. $X^4$ and/or $X^5$ can be hydrogen, which means there is no protecting group on the hydroxyl group.

$X^6$ is a protecting group for the guanidino group of Arg selected from the group consisting of nitro, Tos, CBZ, adamantyloxycarbonyl, and BOC, or is hydrogen;

$X^7$ is hydrogen or a protecting group for the side chain amino substituent of Lys. Illustrative of suitable side chain amino protecting groups are 2-chlorobenzyloxycarbonyl (2-Cl-Z), Tos, CBZ, t-amyloxycarbonyl and BOC. .

The selection of a side chain amino protecting group is not critical except that it must be one which is not removed during deprotection of the α-amino groups during the synthesis. Hence, the α-amino protecting group and the side chain amino protecting group cannot be the same.

$M^8$ is selected from the class consisting of OH, $OCH_3$, esters, amides, hydrazides, —O—$CH_2$-resin support and —NH-resin support, with the groups other than OH and amides being broadly considered as protecting groups.

In the formula for the intermediate, at least one of $X^1$, $X^2$, $X^3$, $X^4$, $X^5$, $X^6$, $X^7$, and $X^8$ is a protecting group.

In selecting a particular side chain protecting group to be used in the synthesis of the peptides, the following rules are followed: (a) the protecting group should be stable to the reagent and under the reaction conditions selected for removing the α-amino protecting group at each step of the synthesis, (b) the protecting group should retain its protecting properties and not be split off under coupling conditions,

and (c) the side chain protecting group should be removable, upon the completion of the synthesis containing the desired amino acid sequence, under reaction conditions that will not alter the peptide chain.

The peptides are preferably prepared using solid phase synthesis, such as that described by Merrifield, *J. Am. Chem. Soc.,* 85, p 2149 (1963), although other equivalent chemical syntheses known in the art can also be used as previously mentioned. Solid-phase synthesis is commenced from the C-terminal end of the peptide by coupling a protected α-amino acid to a suitable resin. Such a starting material can be prepared by attaching α-amino-protected Leu or Ala by an ester linkage to a chloromethylated resin or a hydroxymethyl resin, or by an amide bond to a BHA resin or MBHA resin. The preparation of the hydroxymethyl resin is described by Bodansky et al., *Chem. Ind.* (London) 38, 1597—98 (1966). Chloromethylated resins are commercially available from Bio Rad Laboratories, Richmond, California and from Lab. Systems, Inc. The preparation of such a resin is described by Stewart et al., "Solid Phase Peptide Synthesis" (Freeman & Co., San Francisco 1969), Chapter 1, pp 1—6. BHA and MBHA resin supports are commercially available and are generally used only when the desired polypeptide being synthesized has an α-carboxamide at the C-terminal.

Ala protected by BOC is coupled to the chloromethylated resin according to the procedure of Monahan and Gilon, *Biopolymer* 12, pp 2513—19, 1973 when, for example, it is desired to synthesize the 40-amino acid peptide. Following the coupling of BOC-Ala to the resin support, the α-amino protecting group is removed, as by using trifluoroacetic acid (TFA) in methylene chloride, TFA alone or HCl in dioxane. The deprotection is carried out at a temperature between about 0°C and room temperature. Other standard cleaving reagents and conditions for removal of specific α-amino protecting groups may be used as described in Schroder & Lubke, "The Peptides", 1 pp 72—75 (Academic Press 1965).

After removal of the α-amino protecting group of Ala, the remaining α-amino- and side chain-protected amino acids are coupled step-wise in the desired order to obtain the intermediate compound defined hereinbefore, or as an alternative to adding each amino acid separately in the synthesis, some of them may be coupled to one another prior to addition to the solid phase reactor. The selection of an appropriate coupling reagent is within the skill of the art. Particularly suitable as a coupling reagent is N,N'-dicyclohexyl carbodiimide (DCCI).

The activating reagents used in the solid phase synthesis of the peptides are well known in the peptide art. Examples of suitable activating reagents are: (1) carbodiimides, such as N,N'-diisopropyl carbodiimide, N - ethyl - N' - (3 - dimethylaminopropyl)carbodiimide; (2) cyanamides such as N,N' - dibenzylcyanamide; (3) keteimines; (4) isoxazolium salts, such as N - ethyl - 5 - phenyl isoxazolium - 3' - sulfonate; (5) monocyclic nitrogen-containing heterocyclic amides of aromatic character containing one through four nitrogens in the ring, such as imidazolides, pyrazolides, and 1,2,4 - triazolides, specific heterocyclic amides that are useful include N,N' - carbonyl diimidazole, N,N' - carbonyl - di - 1,2,4 - triazole; (6) alkoxylated acetylene, such as ethoxyacetylene; (7) reagents which form a mixed anhydride with the carboxyl moiety of the amino acid, such as ethylchloroformate and isobutylchloroformate and (8) reagents which form an active ester with the carboxyl moiety of the amino acid, such as nitrogen-containing heterocyclic compounds having a hydroxy group on one ring nitrogen, e.g. N-hydroxyphthalimide, N-hydroxysuccinimide and 1-hydroxybenzotriazole(HOBT). Other activating reagents and their use in peptide coupling are described by Schroder & Lubke supra, in Chapter III and by Kapoor, *J. Phar. Sci.,* 59, pp 1—27 (1970).

Each protected amino acid or amino acid sequence is introduced into the solid phase reactor in about a twofold or more excess, and the coupling may be carried out in a medium of dimethylformamide(DMF):$CH_2Cl_2$ (1:1) or in DMF or $CH_2Cl_2$ alone. In cases where incomplete coupling occurred, the coupling procedure is repeated before removal of the α-amino protecting group prior to the coupling of the next amino acid. The success of the coupling reaction at each stage of the synthesis is monitored by the ninhydrin reaction, as described by E. Kaiser et al., *Anal. Biochem.* 34, 595 (1970).

After the desired amino acid sequence has been completed, the intermediate peptide is removed from the resin support by treatment with a reagent, such as liquid hydrogen fluoride, which not only cleaves the peptide from the resin but also cleaves all remaining side chain protecting groups $X^2$, $X^3$, $X^4$, $X^5$, $X^6$, $X^7$ and $X^8$ and the α-amino protecting group $X^1$, to obtain the peptide.

As an alternative route, the intermediate peptide may be separated from the resin support by alcoholysis after which the recovered C-terminal alkyl ester is converted to the acid by hydrolysis. Any side chain protecting groups may then be cleaved as previously described or by other known procedures, such as catalytic reduction (e.g. Pd on $BaSO_4$). When using hydrogen fluoride for cleaving, anisole and methylethyl sulfide are included in the reaction vessel for scavenging.

The following Example sets forth the preferred method for synthesizing PGRF by the solid-phase technique. It will of course be appreciated that the synthesis of a correspondingly shorter peptide fragment is effected in the same manner by merely eliminating the requisite number of amino acids at either end of the chain; however, it is presently felt that biologically active fragments should contain the indicated sequence at the N-terminal.

Example I

The synthesis of PGRF(1—44) free acid having the formula:

H-Tyr-Ala-Asp-Ala-Ile-Phe-Thr-Asn-Ser-Tyr-Arg-Lys-Val-Leu-
Gly-Gln-Leu-Ser-Ala-Arg-Lys-Leu-Leu-Gln-Asp-Ile-Met-Ser-
Arg-Gln-Gln-Gly-Glu-Ser-Asn-Gln-Glu-Arg-Gly-Ala-Arg-Ala-Arg-Leu-OH

is conducted in a stepwise manner using a Beckman 990 Peptide Synthesizer on a chloromethylated resin, such as that available from Lab Systems, Inc., containing 0.9 Meq Cl/g. Coupling of BOC-Leu to the resin is performed by the general procedure set forth by Monahan et al. in *Biopolymers,* Volume 12 (1973) pp. 2513—2519, and it results in the substitution of about 0.22 mmol. Leu per gram of resin. All solvents that are used are carefully degassed by sparging with an inert gas, preferably helium, to insure the absence of oxygen that might undesirably oxidize the sulfur of the Met residue.

After deprotection and neutralization, the peptide chain is built step-by-step on the resin. Deprotection, neutralization and addition of each amino acid is performed in general accordance with the procedure set forth in detail in Guillemin et al. U.S. Patent No. 3,904,594. The couplings are specifically carried out as set out in the following schedule.

Schedule

| Step | Reagents and operations | Mix times min. |
|---|---|---|
| 1 | $CH_2Cl_2$ wash (2 times) | 0.5 |
| 2 | 50% trifluoroacetic acid (TFA)+5% 1,2-ethanedithiol in $CH_2Cl_2$ (1 time) | 0.5 |
| 3 | 50% trifluoroacetic acid (TFA)+5% 1,2-ethanedithiol in $CH_2Cl_2$ (1 time) | 20.0 |
| 4 | $CH_2Cl_2$ wash (3 times) | 0.5 |
| 5 | $CH_3OH$ wash (2 times) | 0.5 |
| 6 | 10% triethylamine ($Et_3N$) in $CH_2Cl_2$ neutralization (2 times) | 0.5 |
| 7 | $CH_3OH$ wash (2 times) | 0.5 |
| 8 | 10% triethylamine ($Et_3N$) in $CH_2Cl_2$ neutralization (2 times) | 0.5 |
| 9 | $CH_3OH$ wash (2 times) | 0.5 |
| 10 | $CH_2Cl_2$ wash (2 times) | 0.5 |
| 11 | *Boc-amino acid (1 mmole/g resin) plus equivalent amount of dicyclohexylcarbodiimide (DCC) in $CH_2Cl_2$ | 120 |
| 12 | $CH_2Cl_2$ wash (1 time) | 0.5 |
| 13 | 50% dimethylformamide in $CH_2Cl_2$ wash (2 times) | 0.5 |
| 14 | 10% triethylamine ($Et_3N$) in $CH_2Cl_2$ wash (1 time) | 0.5 |
| 15 | $CH_3OH$ wash (2 times) | 0.5 |
| 16 | $CH_2Cl_2$ wash (2 times) | 0.5 |
| 17 | 25% acetic anhydride in $CH_2Cl_2$ (2 ml/g resin) | 20.0 |
| 18 | $CH_2Cl_2$ wash (2 times) | 0.5 |
| 19 | $CH_3OH$ wash (2 times) | 0.5 |

* For the coupling of Asn and Gln, an 1.136 molar excess of 1-hydroxybenzotriazole (HOBt) was included in this step.

Briefly, for the coupling reaction, one mmol. of BOC-protected amino acid in methylene chloride is used per gram of resin, plus one equivalent of 0.5 molar DCCI in methylene chloride or 30% DMF in methylene chloride, for two hours. When Arg is being coupled, a mixture of 10% DMF and methylene chloride is used. Bzl is used as the hydroxyl side-chain protecting group for Ser and Thr. 2-chloro-benzyloxycarbonyl (2Cl-Z) is used as the protecting group for the Lys side chain. Tos is used to protect the guanidino group of Arg, and the Glu or Asp carboxyl group is protected as the Bzl ester. The phenolic hydroxyl group of Tyr is protected with 2,6-dichlorobenzyl. At the end of the synthesis, the following composition is obtained:

$X^1$-Tyr($X^2$)-Ala-Asp($X^3$)-Ala-Ile-Phe-Thr($X^4$)-Asn-Ser($X^5$)-Tyr($X^2$)-Arg($X^6$)-Lys($X^7$)-
Val-Leu-Gly-Gln-Leu-Ser($X^5$)-Ala-Arg($X^6$)-Lys($X^7$)-Leu-Leu-Gln-Asp($X^3$)-Ile-Met-Ser($X^5$)-
Arg($X^6$)-Gln-Gln-Gly-Glu($X^3$)-Ser($X^5$)-Asn-Gln-Glu($X^3$)-Arg($X^6$)-Gly-Ala-Arg($X^6$)-
Ala-Arg($X^6$)-Leu-$X^8$

wherein $X^1$ is BOC, $X^2$ is 2,6-dichlorobenzyl, $X^3$ is benzyl ester, $X^4$ is Bzl, $X^5$ is Bzl, $X^6$ is Tos, $X^7$ is 2Cl-Z and $X^8$ is —O—$CH_2$-benzene-polystyrene resin support.

After the final Tyr residue has been coupled to the resin, the BOC group is removed with 45% TFA in $CH_2Cl_2$. In order to cleave and deprotect the remaining protected peptide-resin, it is treated with 1.5 ml. anisole, 0.25 ml. methylethylsulfide and 10 ml. hydrogen fluoride (HF) per gram of peptide-resin, at −20°C. for one-half hour and at 0°C. for one-half hour. After elimination of the HF under high vacuum, the resin-peptide remainder is washed alternately with dry diethyl ether and chloroform, and the peptide is then extracted with degassed 2N aqueous acetic acid. Lyophilization of the acetic acid extract provides a white fluffy material.

The cleaved and deprotected peptide is then dissolved in 30% acetic acid and subjected to Sephadex® G-50 fine gel filtration.

The peptide is then further purified by CM-32 carboxymethyl cellulose (Whatman) cation-exchange chromatography (1.8×18 cm., $V_{bed}$=50 ml.) using a concave gradient generated by dropping 1 L. of 0.4 M $NH_4OAc$, pH 6.5 into a mixing flask containing 400 ml. 0.01 M. $NH_4OAc$, pH 4.5. Final purification is carried out using partition chromatography on Sephadex® G-50 fine support (Pharmacia) with a nBuOH:EtOH:pyridine:0.2% NHOAc (4:1:1:7) solvent system. Purification details are generally set forth in Ling et al. *Biochem. Biophys. Res. Commun.* 95, 945 (1980). The chromatographic fractions are carefully monitored by TLC, and only the fractions showing substantial purity were pooled.

Amino acid analysis is carried out following hydrolysis in sealed tubes using methodology as described in *Anal. Biochem., 126,* 144—156 (1982) using a Liquimat III amino acid analyzer to check the correct sequence was obtained, giving the following results: Asx(3.62), Thr(0.75), Ser(3.5), Glx(6.83), Gly(2.87), Ala(5.10), Val(0.9), Met(1.23), Ile(1.84), Leu(5.045), Tyr(2.09), Phe(0.91), Lys(2.31), and Arg(6.61). Analysis confirmed the correct sequence.

Example II

The synthesis of PGRF(1—40) having the formula:

H-Tyr-Ala-Asp-Ala-Ile-Phe-Thr-Asn-Ser-Tyr-Arg-Lys-Val-Leu-
Gly-Gln-Leu-Ser-Ala-Arg-Lys-Leu-Leu-Gln-Asp-Ile-Met-Ser-
Arg-Gln-Gln-Gly-Glu-Ser-Asn-Gln-Glu-Arg-Gly-Ala-OH

is conducted in a stepwise manner using a Beckman 990 Peptide Synthesizer on a chloromethylated resin in the manner described in Example I. The peptide is judged to be substantially pure using TLC and HPLC.

Amino acid analysis is carried out following hydrolysis to check the correct sequence was obtained, giving the following results: Asx(3.89), Thr(0.88), Ser(3.66), Glx(7.04), Gly(3.07), Ala(4.02), Val(0.96), Met(1.01), Ile(1.86), Leu(4.28), Tyr(2.0), Phe(0.86), Lys(2.24), and Arg(4.15). Analysis confirmed the correct sequence.

Example IIA

The synthesis of PGRF(1—34)-free acid having the formula:

H-Tyr-Ala-Asp-Ala-Ile-Phe-Thr-Asn-Ser-Tyr-Arg-Lys-Val-Leu-
Gly-Gln-Leu-Ser-Ala-Arg-Lys-Leu-Leu-Gln-Asp-Ile-Met-Ser-
Arg-Gln-Gln-Gly-Glu-Ser-OH

is conducted in a stepwise manner using a Beckman 990 Peptide Synthesizer on a chloromethylated resin in the manner described in Example I. The peptide is judged to be substantially pure using TLC and HPLC.

Amino acid analysis is carried out following hydrolysis to check the correct sequence was obtained, giving the following results: Asx(2.87), Thr(0.78), Ser(3.78), Glx(5.11), Gly(1.93), Ala(3.03), Val(0.88), Met(0.96), Ile(1.88), Leu(4.14), Tyr(2.05), Phe(1.07), Lys(2.29), and Arg(3.22). Analysis confirmed the correct sequence.

Example IIB

The synthesis of PGRF(1—31)-free acid having the formula:

H-Tyr-Ala-Asp-Ala-Ile-Phe-Thr-Asn-Ser-Tyr-Arg-Lys-Val-Leu-
Gly-Gln-Leu-Ser-Ala-Arg-Lys-Leu-Leu-Gln-Asp-Ile-Met-Ser-
Arg-Gln-Gln-OH

is conducted in a stepwise manner using a Beckman 990 Peptide Synthesizer on a chloromethylated resin in the manner described in Example I. The peptide is judged to be substantially pure using TLC and HPLC.

Amino acid analysis is carried out following hydrolysis to check the correct sequence was obtained, giving the following results: Asx(2.73), Thr(0.75), Ser(2.77), Glx(3.95), Gly(0.98), Ala(3.06), Val(0.80), Met(0.98), Ile(1.77), Leu(4.28), Tyr(2.23), Phe(1.14), Lys(2.34), and Arg(3.22). Analysis confirmed the correct sequence.

Example IIC

The synthesis of PGRF(1—28)-free acid having the formula:

H-Tyr-Ala-Asp-Ala-Ile-Phe-Tyr-Asn-Ser-Tyr-Arg-Lys-Val-Leu-
Gly-Gln-Leu-Ser-Ala-Arg-Lys-Leu-Leu-Gln-Asp-Ile-Met-Ser-OH

is conducted in a stepwise manner using a Beckman 990 Peptide Synthesizer on a chloromethylated resin in the manner described in Example I. The peptide is judged to be substantially pure using TLC and HPLC.

Amino acid analysis is carried out following hydrolysis to check the correct sequence was obtained, giving the following results: Asx(2.66), Thr(0.73), Ser(2.66), Glx(1.98), Gly(0.81), Ala(2.89), Val(0.90), Met(1.15), Ile(1.72), Leu(4.14), Tyr(2.43), Phe(1.58), Lys(2.15), and Arg(2.19). Analysis confirmed the correct sequence.

Example III

The synthesis of PGRF(1—44)-amide having the formula:

H-Tyr-Ala-Asp-Ala-Ile-Phe-Thr-Asn-Ser-Tyr-Arg-Lys-Val-Leu-
Gly-Gln-Leu-Ser-Ala-Arg-Lys-Leu-Leu-Gln-Asp-Ile-Met-Ser-
Arg-Gln-Gln-Gly-Glu-Ser-Asn-Gln-Glu-Arg-Gly-Ala-Arg-Ala-Arg-Leu-$NH_2$

is conducted in a stepwise manner using a Beckman 990 Peptide Synthesizer on an MBHA resin in the manner described in Example I. This peptide is judged to be substantially pure using TLC and HPLC.

Amino acid analysis is carried out following hydrolysis to check the correct sequence was obtained, giving the following results: Asx(3.75), Thr(0.80), Ser(3.60), Glx(6.98), Gly(3.16), Ala(5.04), Val(0.77), Met(1.02), Ile(1.79), Leu(5.41), Tyr(2.03), Phe(0.84), Lys(2.39), and Arg(6.43). Analysis confirmed the correct sequence.

Example IV

The synthesis of a PGRF analog having the formula:

H-Tyr-Ala-Asp-Ala-Ile-Phe-Thr-Asn-Ser-Tyr-Arg-Lys-Val-Leu-
Gly-Gln-Leu-Ser-Ala-Arg-Lys-Leu-Leu-Gln-Asp-Ile-Met-Ser-
Arg-Gln-Gln-Gly-Glu-Ala-Asn-Gln-Glu-Ser-Gly-Arg-OH

is conducted in a stepwise manner using a Beckman 990 Peptide Synthesizer on a chloromethylated resin, such as that available from Lab Systems, Inc. in the manner described in Example I. The peptide is judged to be substantially pure using TLC and HPLC.

Amino acid analysis is carried out following hydrolysis to check the correct sequence was obtained, giving the following results: Asx(4.35), Thr(1.06), Ser(3.80), Glx(7.53), Gly(2.96), Ala(4.05), Val(0.97), Met(0.86), Ile(1.94), Leu(3.70), Tyr(2.05), Phe(1.06), Lys(2.06), and Arg(3.53). Analysis confirmed the correct sequence.

Example V

The synthesis of a PGRF(1—40)-amide having the formula:

H-Tyr-Ala-Asp-Ala-Ile-Phe-Thr-Asn-Ser-Tyr-Arg-Lys-Val-Leu-
Gly-Gln-Leu-Ser-Ala-Arg-Lys-Leu-Leu-Gln-Asp-Ile-Met-Ser-
Arg-Gln-Gln-Gly-Glu-Ser-Asn-Gln-Glu-Arg-Gly-Ala-$NH_2$

is conducted in a stepwise manner using a Beckman 990 Peptide Synthesizer on an MBHA resin in the manner described in Example I. This peptide is judged to be substantially pure using TLC and HPLC.

Amino acid analysis is carried out following hydrolysis to check the correct sequence was obtained, giving the following results: Asx(3.76), Thr(0.88), Ser(3.68), Glx(6.89), Gly(3.12), Ala(4.08), Val(0.88), Met(1.36), Ile(1.76), Leu(4.24), Tyr(2.00), Phe(0.80), Lys(2.32), and Arg(4.16). Analysis confirmed the correct sequence.

Example VI

The synthesis of PGRF(1—37)-free acid having the formula:

H-Tyr-Ala-Asp-Ala-Ile-Phe-Thr-Asn-Ser-Tyr-Arg-Lys-Val-Leu-
Gly-Gln-Leu-Ser-Ala-Arg-Lys-Leu-Leu-Gln-Asp-Ile-Met-Ser-
Arg-Gln-Gln-Gly-Glu-Ser-Asn-Gln-Glu-OH

is conducted in a stepwise manner using a Beckman 990 Peptide Synthesizer on a chloromethylated resin in the manner described in Example I. The peptide is judged to be substantially pure using TLC and HPLC.

Amino acid analysis is carried out following hydrolysis to check the correct sequence was obtained, giving the following results: Asx(3.92), Thr(0.79), Ser(3.62), Glx(7.05), Gly(1.97), Ala(3.17), Val(1.03), Met(1.0), Ile(1.91), Leu(4.37), Tyr(1.86), Phe(0.76), Lys(2.15), and Arg(3.40). Analysis confirmed the correct sequence.

Example VIA

The synthesis of PGRF(1—37)-amide having the formula:

H-Tyr-Ala-Asp-Ala-Ile-Phe-Thr-Asn-Ser-Tyr-Arg-Lys-Val-Leu-
Gly-Gln-Leu-Ser-Ala-Arg-Lys-Leu-Leu-Gln-Asp-Ile-Met-Ser-
Arg-Gln-Gln-Gly-Glu-Ser-Asn-Gln-Glu-$NH_2$

is conducted in a stepwise manner using a Beckman 990 Peptide Synthesizer on an MBHA resin in the manner described in Example I. The peptide is judged to be substantially pure using TLC and HPLC.

Amino acid analysis is carried out following hydrolysis to check the correct sequence was obtained, giving the following results: Asx(4.02), Thr(0.80), Ser(3.49), Glx(6.90), Gly(1.92), Ala(3.08), Val(1.05), Met(1.01), Ile(1.77), Leu(4.05), Tyr(2.02), Phe(1.14), Lys(2.50), and Arg(3.27). Analysis confirmed the correct sequence.

Example VII

To determine the effectiveness of the peptides to promote the release of growth hormone, *in vitro* assays are carried out using synthetic PGRF(1—40) of Example II in side-by-side comparison with equimolar concentrations of the extracted and purified native PGRF (1—40) and of a GRF Reference Standard having a known effectiveness to promote the release of growth hormone from pituitary cells. The GRF Reference standard is described and defined in Brazeau, et al., *Endocrinology,* Vol. 110, A538(1982) and is an amount of a preparation of rat hypothalamic origin that produces a half-maximal response in terms of GH release in a pituitary cell monolayer bioassay. Cultures are used which include cells of rat pituitary glands removed some four to five days previously. Both cultures of a defined standard medium and cultures which are considered optimal for the secretion of growth hormone are used for the comparative testing, in the general manner described in Brazeau, et al. *Regulatory Peptides,* 1, 255, 1981. Incubation with the substance to be tested is carried out for 3 to 4 hours, and aliquots of the culture medium are removed and processed to measure their contents in immunoreactive GH(ir GH) by a well-characterized radioimmunoassay.

The results of this comparative testing shows that, in equimolar ratios, the synthetic PGRF (1—40) has the full biological activity of the native peptide, as shown in Table I. The $ED_{50}$ of the synthetic peptide is about 113 picograms, which is far more potent than any other molecule heretofore claimed as a GH releasing factor.

TABLE I

| GRF Reference Standard | GH secretion *in vitro* % of controls |
|---|---|
| 0.63 unit | 173±0.4 |
| 1.25 units | 230±5 |
| 2.50 units | 347±13 |
| 5.00 units | 474±3 |
| 10.00 units | 674±6 |
| Native PGRF(1—40) 12.5 femtomoles | 234±17 |
| 25 fmoles | 351±7 |
| 50 fmoles | 528±16 |
| 100 fmoles | 720±32 |
| 200 fmoles | 748±7 |
| Synthetic PGRF(1—40) 10 fmoles | 269±20 |
| 100 fmoles | 701±6 |
| 1000 fmoles | 990±42 |

In addition to the *in vitro* tests for secretion of growth hormone, *in vivo* experiments were also run by injecting the synthetic peptide into normal male rats about 200 g. body weight, anesthetized with pentobarbital. The results set forth in Table II show that the synthetic PGRF peptide is a powerful stimulator of the secretion of pituitary growth hormone.

TABLE II

*in vivo* effects of synthetic PGRF(1-40) on the release of pituitary growth hormone following one single intravenous injection in normal rats (4 animals per treatment dose).

| | Responses in serum ir-GH in nanograms/ml at indicated times before and after injection. | | | | | |
|---|---|---|---|---|---|---|
| Doses | −1 min | +5 min | +10 min | +15 min | +30 min | +60 min |
| 0 microgram | 173±47 | 251±81 | 339±139 | 396±121 | 749±440 | 316±76 |
| 0.01 μg | 173±23 | 284±20 | 238±51 | 201±47 | 261±50 | 299±25 |
| 0.1 μg | 276±126 | 694±246 | 582±290 | 758±562 | 280±70 | 424±129 |
| 1.0 μg | 142±24 | 4551±1825 | 1748±564 | 730±158 | 234±53 | 267±129 |
| 10.0 μg | 234±76 | 7077±1943 | 4676±585 | 2464±378 | 616±112 | 223±26 |

Additional testing shows that synthetic PGRF analog from Example IV exhibits substantially the same biological potency as the native PGRF(1—40) and that the synthetic fragments from Examples VI and VIA also exhibit very substantial biological potency. Furthermore the PGRF(1—40) peptide having the α-carboxamide at the C-terminal from Example V has substantially twice the biological potency of the synthetic peptide tested in Example VII.

Example VIII

The *in vitro* assays described in Example VII are repeated using Native PGRF(1—40) and Native PGRF(1—44), and the results show that Native PGRF(1—44) has more than about twice the biological potency.

Further testing shows that synthetic PGRF(1—44) free acid as synthesized in Example I exhibits somewhat less potency than native PGRF(1—44) and that synthetic PGRF(1—44)-amide exhibits substantially the same potency as native PGRF(1—44). Synthetic PGRF(1—44)-amide, when injected in laboratory animals(rats), shows the same type of GH-releasing activity as exhibited by PGRF(1—40) in Table II of Example VII, but is about 2.5 to 3.0 times more potent on a weight basis than PGRF(1—40)-free acid.

Approximately 1 out of 7000 to 15,000 children born in the USA are known to be pituitary growth hormone deficient or "pituitary-dwarfs", i.e., they are dwarfs because they lack the normal levels of pituitary GH in their blood. There are clinical reasons to propose that most of these patients have a normal pituitary gland and that the cause of their problem is a lack either of the synthesis, or of the secretion, of the hypothalamic releasing factor for GH. Synthetic PGRF is expected to be the ideal treatment for these cases who have heretofore been treated by injections of human pituitary GH, an extremely expensive preparation obtained exclusively from human pituitaries at autopsies. Human GH prepared by DNA-recombinant methodology, though announced in the literature, is not currently available for routine use. Synthetic PGRF is a far simpler molecule and should have significant advantages for use throughout the world where the number of such pituitary dwarfs is estimated to be several hundreds of thousands.

Because synthetic PGRF is the first known molecule specifically to assess the pituitary function in terms of GH secretion, it thus represents the first routine test for GH secretion in all cases in which a specific defect of pituitary function is suspected by a physician. Synthetic PGRF should henceforth replace the cumbersome methods used currently (arginine infusions, hypoglycemia, L-DOPA injections, etc.) to assess GH secretory ability as a diagnostic procedure.

Synthetic PGRF should be of interest in all cases in clinical medicine in which a physician wishes to favor a positive nitrogen balance and anabolism, such as wound-healing, treatment of extensive burns, post-operative periods following extensive surgery and other medical situations of debilitation, including many syndromes of gerontological practice as well as of the pediatric practice of prematurely born infants. Stimulation of GH secretion is of interest in patients during and after extensive radiation therapy for solid tumors, again to promote anabolism and also to take advantage of the effects of GH on the stimulation of the stem cells of the hematopoietic system. For administration to humans, synthetic PGRF peptides should have a purity of at least about 93% and preferably at least 98%. This purity means the intended peptide constitutes the stated weight % of all like peptides and peptide fragments present.

Most of the biologically active peptides have been found to possess biological activities other than those for which they were originally recognized. In view of such precedents, it is likely that PGRF will be found to possess extrapituitary activities which may be of practical interest. Although PGRF was extracted and isolated from a human pancreatic tumor, on the basis of overall experience and experimentation, it is believed the amino acid sequence of PGRF(1—44)-amide is the same as the sequence of human hypothalamic GH releasing factor.

Chronic administration of synthetic PGRF peptides to farm animals or other warm-blooded animals is expected to promote anabolism and thus increase body weight in terms of muscle mass. Use in aquiculture for raising fish and other cold-blooded marine animals to accelerate growth is also intended. Administration to animals at a purity as low as about 5% may be acceptable.

Synthetic PGRF or the nontoxic salts thereof, combined with a pharmaceutically acceptable carrier to form a pharmaceutical composition, may be administered to mammals, including humans, either intravenously, subcutaneously, intramuscularly or orally. The administration may be employed by a physician to stimulate the release of growth hormone where the host being treated requires such therapeutic treatment. The required dosage will vary with the particular condition being treated, with the severity of the condition and with the duration of desired treatment.

Such peptides are often administered in the form of pharmaceutically acceptable nontoxic salts, such as acid addition salts or metal complexes, e.g., with zinc, iron or the like (which are considered as salts for purposes of this application). Illustrative of such acid addition salts are hydrochloride, hydrobromide, sulphate, phosphate, maleate, acetate, citrate, benzoate, succinate, malate, ascorbate, tartrate and the like. If the active ingredient is to be administered in tablet form, the tablet may contain a binder, such as tragacanth, corn starch or gelatin; a disintegrating agent, such as alginic acid; and a lubricant, such as magnesium stearate. If administration in liquid form is desired, sweetening and/or flavoring may be used, and intravenous administration in isotonic saline, phosphate buffer solutions or the like may be effected.

The peptides should be administered under the guidance of a physician, and pharmaceutical compositions will usually contain the peptide in conjunction with a conventional, pharmaceutically-acceptable carrier. Usually, the dosage will be from about 20 to about 2000 nanograms of the peptide per kilogram of the body weight of the host.

Although the invention has been described with regard to its preferred embodiments, which constitute the best mode presently known to the inventors, it should be understood that various changes and modifications as would be obvious to one having the ordinary skill in this art may be made without

departing from the scope of the invention which is set forth in the claims appended hereto. For example, modifications in the 44-member chain, particularly deletions beginning at the carboxyl terminal of the peptide, can be made in accordance with the known experimental practises to date to create fragments less than 40 residues in length, e.g. PGRF(1—27), and having either $NH_2$ or OH at the C-terminal that retain all or very substantial portions of the potency of the peptide, and such peptides are considered as being within the scope of the invention. Moreover, additions can be made to either terminal, or to both terminals, and/or generally equivalent residues can be substituted for naturally occurring residues, as is well-known in the overall art of peptide chemistry to produce analogs having at least a substantial portion of the potency of the native polypeptide without deviating from the scope of the invention.

**Claims for the Contracting States: BE, CH, DE, FR, GB, IT, LI, LU, NL, SE**

1. A synthetic peptide having the formula:

H-Tyr-Ala-Asp-Ala-Ile-Phe-Thr-Asn-Ser-Tyr-Arg-Lys-
Val-Leu-Gly-Gln-Leu-Ser-Ala-Arg-Lys-Leu-Leu-Gln-Asp-
Ile-Met-Ser-Arg-Gln-Gln-Gly-Glu-$R_{34}$-Asn-Gln-Glu-$R_{38}$-Gly-$R_{40}$-$R_{41}$-R

wherein R is OH or $NH_2$, $R_{34}$ is Ser or Ala, $R_{38}$ is Arg or Ser, $R_{40}$ is Ala or Arg and $R_{41}$ is Arg, Arg-Ala, Arg-Ala-Arg, Arg-Ala-Arg-Leu or des-$R_{41}$, or a biologically active fragment thereof, or a nontoxic salt thereof.

2. A synthetic peptide having the formula of Claim 1 wherein $R_{34}$ is Ser, $R_{38}$ is Arg and $R_{40}$ is Ala.

3. A synthetic peptide having the formula of Claim 1 wherein $R_{34}$ is Ala, $R_{38}$ is Ser and $R_{40}$ is Arg.

4. A synthetic peptide having the formula of any one of Claims 1 to 3 wherein $R_{41}$ is Arg-Ala-Arg-Leu.

5. A synthetic peptide having the formula of any one of Claims 1 to 4 wherein R is $NH_2$.

6. A synthetic peptide having the formula of any one of Claims 1 to 4 wherein R is OH.

7. The peptide of Claim 1 having the formula:

H-Tyr-Ala-Asp-Ala-Ile-Phe-Thr-Asn-Ser-Tyr-Arg-Lys-
Val-Leu-Gly-Gln-Leu-Ser-Ala-Arg-Lys-Leu-Leu-Gln-Asp-Ile-Met-OH.

8. A method of improving body weight in terms of muscle mass in warm-blooded non-human animals in agriculture wherein an effective amount of a compound as defined in any one of Claims 1 to 7 is administered to stimulate the release of GH to promote growth of the animal and not for any therapeutic purpose.

9. A method of improving aquiculture by administering to cold-blooded animals an amount of a compound as defined in any one of Claims 1 to 7 effective to accelerate the growth thereof and not for any therapeutic purpose.

10. A pharmaceutical composition for stimulating the release of GH in a human comprising a compound as defined in any one of Claims 1 to 7 and a pharmaceutically acceptable liquid or solid carrier therefor.

11. A veterinary composition for stimulating the release of GH in a non-human animal comprising a compound as defined in any one of Claims 1 to 7 and a veterinarily acceptable liquid or solid carrier therefor.

12. A compound in accordance with any one of Claims 1 to 7 for use in stimulating the release of GH in humans.

13. A compound in accordance with any one of Claims 1 to 7 for use in accelerating the growth of warm-blooded non-human animals.

14. A compound in accordance with any one of Claims 1 to 7 for use in aquiculture to accelerate the growth of cold-blooded animals.

15. A biologically active fragment as defined in Claim 1 characterized by the presence of an amino acid sequence:—

H-Tyr-Ala-Asp-Ala-Ile-Phe-Thr-Asn-Ser-Tye-Arg-Lys-Val-
Leu-Gly-Gln-Leu-Ser-Ala-Arg-Lys-Leu-Leu-Gln-Asp-Ile-Met-.

16. A peptide as claimed in Claim 15 and having any of the following formulae:—

(a) H-Tyr-Ala-Asp-Ala-Ile-Phe-Thr-Asn-Ser-Tyr-Arg-Lys-
Val-Leu-Gly-Gln-Leu-Ser-Ala-Arg-Lys-Leu-Leu-Gln-Asp-Ile-
Met-OH.

(b) H-Tyr-Ala-Asp-Ala-Ile-Phe-Thr-Asn-Ser-Tyr-Arg-Lys-
Val-Leu-Gly-Gln-Leu-Ser-Ala-Arg-Lys-Leu-Leu-Gln-Asp-
Ile-Met-Ser-Arg-Gln-Gln-Gly-Glu-Ser-Asn-Gln-Glu-Arg-
Gly-Ala-Arg-Ala-Arg-Leu-OH.

(c) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-OH.

(d) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-OH.

(e) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-OH.

(f) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-OH.

(g) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-Arg-Ala-Arg-Leu-$NH_2$.

(h) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ala-Asn-Gln-Glu-Ser-Gly-Arg-OH.

(i) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-$NH_2$.

(j) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-OH.

(k) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-$NH_2$.

17. A non-toxic salt of a peptide as claimed in any one of Claims 2 to 7, 15 and 16.

**Claims for the Contracting State: AT**

1. A process for the manufacture of compounds defined by the formula (I):

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-$R_{34}$-Asn-Gln-Glu-$R_{38}$-Gly-$R_{40}$-$R_{41}$-R

or biologically active fragments thereof, wherein R is OH or $NH_2$, $R_{34}$ is Ser or Ala, $R_{38}$ is Arg or Ser, $R_{40}$ is Ala or Arg and $R_{41}$ is Arg, Arg-Ala, Arg-Ala-Arg, Arg-Ala-Arg-Leu or des-$R_{41}$ comprising (a) forming a compound having at least one protective group and the formula (II):

$X^1$-Tyr($X^2$)-Ala-Asp($X^3$)-Ala-Ile-Phe-Thr($X^4$)-Asn-Ser($X^5$)-Tyr($X^2$)-Arg($X^6$)-Lys($X^7$)-Val-Leu-Gly-Gln-Leu-Ser($X^5$)-Ala-Arg($X^6$)-Lys($X^7$)-Leu-Leu-Gln-Asp($X^3$)-Ile-Met-Ser($X^5$)-Arg($X^6$)-Gln-Gln-Gly-Glu($X^3$)-$R_{34}$($X^5$)-Asn-Gln-Glu($X^3$)-$R_{38}$($X^5$ or $X^6$)-Gly-$R_{40}$($X^6$)-$R_{41}$($X^6$)-$X^8$,

wherein: ($X^1$), ($X^2$), ($X^3$), ($X^4$), ($X^5$), ($X^6$) and ($X^7$) are each either hydrogen or a protective group and ($X^8$) is either amide or hydroxyl or a protective group, and (b) splitting off the protective group or groups from said compound of the formula (II) to provide a peptide having the formula (I) or a biologically active fragment thereof and, if desired, (c) converting the resulting peptide into a nontoxic addition salt thereof.

2. A process in accordance with Claim 1 wherein $R_{34}$ is Ser, $R_{38}$ is Arg and $R_{40}$ is Ala.

3. A process in accordance with either Claim 1 or 2 wherein $R_{41}$ is Arg-Ala-Arg-Leu.

4. A process in accordance with any one of Claims 1 to 3 wherein R is $NH_2$.

5. A process in accordance with any one of Claims 1 to 3 wherein R is OH.

6. The process in accordance with Claim 1 wherein the formula of the compound is

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-OH.

7. A process in accordance with any one of Claims 1 to 6 wherein $X^1$ is hydrogen or an α-amino protecting group; $X^2$ is hydrogen or a protecting group for the phenolic hydroxyl group of Tyr; $X^3$ is hydrogen or a protecting group for the carboxyl group Asp or Glu; $X^4$ is hydrogen or a protecting group for

the alcoholic hydroxyl group of Thr; $X^5$ is hydrogen or a protecting group for the alcoholic hydroxyl group of Ser; $X^6$ is hydrogen or a protecting group for the guanidino group of Arg; $X^7$ is hydrogen or a protecting group for the side chain amino group of Lys; and $X^8$ is selected from the class consisting of OH, $OCH_3$, esters, amides, hydrazides, —O—$CH_2$-resin support and —NH-resin support.

8. A process in accordance with Claim 7 wherein $X^1$ is BOC, $X^2$ is 2,6-dichlorobenzyl, $X^3$ is Bzl, $X^4$ is Bzl, $X^5$ is Bzl, $X^6$ is Tos, $X^7$ is 2-chlorobenzyloxycarbonyl and $X^8$ is —O—$CH_2$-resin support.

9. A process in accordance with Claim 7 wherein: $X^1$ is BOC, $X^2$ is 2,6-dichlorobenzyl, $X^3$ is Bzl, $X^4$ is Bzl, $X^5$ is Bzl, $X^6$ is Tos, $X^7$ is 2-chlorobenzyloxycarbonyl and $X^8$ is —NH-resin support.

10. A method of improving body weight in terms of muscle mass in warm-blooded non-human animals in agriculture wherein an effective amount of a compound of the formula (I) defined in claim 1 is administered to stimulate the release of GH to promote growth of the animal and not for any therapeutic purpose.

11. A method of improving aquiculture by administering to cold-blooded animals an amount of a compound of the formula (I) defined in Claim 1 effective to accelerate the growth thereof and not for any therapeutic purpose.

**Patentansprüche für die Vertragsstaaten: BE, CH, DE, FR, GB, IT, LI, LU, NL, SE**

1. Synthetisches Peptid der Formel:

H-Tyr-Ala-Asp-Ala-Ile-Phe-Thr-Asn-Ser-Tyr-Arg-Lys-Val-Leu-Gly-Gln-Leu-
Ser-Ala-Arg-Lys-Leu-Leu-Gln-Asp-Ile-Met-Ser-Arg-Gln-Gln-Gly-Glu-$R_{34}$-
Asn-Gln-Glu-$R_{38}$-Gly-$R_{40}$-$R_{41}$-R,

worin R OH oder $NH_2$, $R_{34}$ Ser oder Ala, $R_{38}$ Arg oder Ser, $R_{40}$ Ala oder Arg und $R_{41}$ Arg, Arg-Ala, Arg-Ala-Arg, Arg-Ala-Arg-Leu oder des-$R_{41}$ ist, oder ein biologisch aktiver Rest hiervon oder ein nicht toxisches Salz hiervon.

2. Synthetisches Peptid der Formel aus Anspruch 1, worin $R_{34}$ Ser, $R_{38}$ Arg und $R_{40}$ Ala ist.

3. Synthetisches Peptid der Formel aus Anspruch 1, worin $R_{34}$ Ala, $R_{38}$ Ser und $R_{40}$ Arg ist.

4. Synthetisches Peptid der Formel aus einem der Ansprüche 1 bis 3 worin $R_{41}$ Arg-Ala-Arg-Leu ist.

5. Synthetisches Peptid der Formel aus einem der Ansprüche 1 bis 4, worin R $NH_2$ ist.

6. Synthetisches Peptid der Formel aus einem der Ansprüche 1 bis 4, worin R OH ist.

7. Peptid nach Anspruch 1 der Formel

H-Tyr-Ala-Asp-Ala-Ile-Phe-Thr-Asn-Ser-Tyr-Arg-Lys-Val-Leu-Gly-Gln-Leu-
Ser-Ala-Arg-Lys-Leu-Leu-Gln-Asp-Ile-Met-OH.

8. Verfahren zur Erhöhung des Körpergewichtes über die Muskelmasse bei warmblütigen nicht menschlichen Tieren in der Landwirtschaft, wobei eine wirksame Menge einer Verbindung nach einem der Ansprüche 1 bis 7 zur Anregung der Freisetzung von STH zur Förderung des Wachstums des Tieres und nicht zu einem therapeutischen Zweck verabreicht wird.

9. Verfahren zur Verbesserung der Wassertierzucht durch Verabreichung einer Verbindung nach einem der Ansprüche 1 bis 7 an kaltblütige Tiere in einer zur Beschleunigung des Wachstums derselben wirksamen Menge und nicht zu einem therapeutischen Zweck.

10. Pharmazeutische Zusammensetzung zur Anregung der Freisetzung von STH bei einem Menschen, welche eine Verbindung nach einem der Ansprüche 1 bis 7 und ein pharmazeutisch annehmbares flüssiges oder festes Trägermaterial dafür enthält.

11. Tiermedizinische Zusammensetzung zur Anregung der Freisetzung von STH bei einem nicht menschlichen Tier, welche eine Verbindung nach einem der Ansprüche 1 bis 7 und ein tiermedizinisch annehmbares flüssiges oder festes Trägermaterial dafür enthält.

12. Verbindung nach einem der Ansprüche 1 bis 7 zur Anwendung in der Anregung der Freisetzung von STH bei Menschen.

13. Verbindung nach einem der Ansprüche 1 bis 7 zur Anwendung in der Beschleunigung des Wachstums von warmblütigen nicht menschlichen Tieren.

14. Verbindung nach einem der Ansprüche 1 bis 7 zur Anwendung in der Wassertierzucht zur Beschleunigung des Wachstums kaltblütiger Tiere.

15. Biologisch wirksamer Rest nach Anspruch 1, gekennzeichnet durch die Anwesenheit einer Aminosäuresequenz:

H-Tyr-Ala-Asp-Ala-Ile-Phe-Thr-Asn-Ser-Tyr-Arg-Lys-Val-Leu-Gly-Gln-Leu-
Ser-Ala-Arg-Lys-Leu-Leu-Gln-Asp-Ile-Met-.

16. Peptid nach Anspruch 15 und einer der folgenden Formeln:

(a) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-OH.
(b) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-Arg-Ala-Arg-Leu-OH.
(c) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-OH.
(d) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-OH.
(e) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-OH.
(f) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-OH.
(g) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-Arg-Ala-Arg-Leu-$NH_2$.
(h) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ala-Asn-Gln-Glu-Ser-Gly-Arg-OH.
(i) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-$NH_2$.
(j) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-OH.
(k) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-$NH_2$.

17. Nicht toxisches Salz eines Peptides nach einem der Ansprüche 2 bis 7, 15 und 16.

**Patentansprüche für den Vertragsstaat: AT**

1. Verfahren zur Herstellung von Verbindungen der Formel (I):

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-$R_{34}$-Asn-Gln-Glu-$R_{38}$-Gly-$R_{40}$-$R_{41}$-R,

oder biologisch wirksamer Reste hiervon, worin R OH oder $NH_2$, $R_{34}$ Ser oder Ala, $R_{38}$ Arg oder Ser, $R_{40}$ Ala oder Arg und $R_{41}$ Arg, Arg-Ala, Arg-Ala-Arg, Arg-Ala-Arg-Leu oder des-$R_{41}$ ist, welches (a) Bildung einer Verbindung mit wenigstens einer Schutzgruppe und der Formel (II):

$X^1$-Tyr($X^2$)-Ala-Asp($X^3$)-Ala-Ile-Phe-Thr($X^4$)-Asn-Ser($X^5$)-Tyr($X^2$)-Arg($X^6$)-Lys($X^7$)-Val-Leu-Gly-Gln-Leu-Ser($X^5$)-Ala-Arg($X^6$)-Lys($X^7$)-Leu-Leu-Gln-Asp($X^3$)-Ile-Met-Ser($X^5$)-Arg($X^6$)-Gln-Gln-Gly-Glu($X^3$)-$R_{34}$($X^5$)-Asn-Gln-Glu($X^3$)-$R_{38}$ ($X^5$ oder $X^6$)-Gly-$R_{40}$($X^6$)-$R_{41}$($X^6$)-$X^8$,

worin ($X^1$), ($X^2$), ($X^3$), ($X^4$), ($X^5$), ($X^6$) und ($X^7$) entweder Wasserstoff oder eine Schutzgruppe und ($X^8$) entweder Amid oder Hydroxy oder eine Schutzgruppe ist, und (b) Abspalten der Schutzgruppe oder -gruppen von dieser Verbindung der Formel (II) zur Herstellung eines Peptides der Formel (I) oder eines biologisch wirksamen Restes hiervon und gewünschtenfalls (c) Umsetzung des sich ergebenden Peptides zu einem nicht toxischen Salz hiervon umfaßt.

2. Verfahren nach Anspruch 1, worin $R_{34}$ Ser, $R_{38}$ Arg und $R_{40}$ Ala ist.

3. Verfahren nach Anspruch 1 oder 2, worin $R_{41}$ Arg-Ala-Arg-Leu ist.

4. Verfahren nach einem der Ansprüche 1 bis 3, worin R $NH_2$ ist.
5. Verfahren nach einem der Ansprüche 1 bis 3, worin R OH ist.
6. Verfahren nach Anspruch 1, worin die Formel der Verbindung

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-OH ist.

7. Verfahren nach einem der Ansprüche 1 bis 6, worin $X^1$ Wasserstoff oder eine α-Amino-Schutzgruppe, $X^2$ Wasserstoff oder eine Schutzgruppe für die phenolische Hydroxygruppe von Tyr, $X^3$ Wasserstoff oder eine Schutzgruppe für die Carboxygruppe von Asp oder Glu, $X^4$ Wasserstoff oder eine Schutzgruppe für die alkoholische Hydroxygruppe von Thr, $X^5$ Wasserstoff oder eine Schutzgruppe für die alkoholische Hydroxygruppe von Ser, $X^6$ Wasserstoff oder eine Schutzgruppe für die Guanidinogruppe von Arg, $X^7$ Wasserstoff oder eine Schutzgruppe für die Seitenkettenaminogruppe von Lys ist und $X^8$ aus der aus OH, $OCH_3$, Estern, Amiden, Hydraziden, —O—$CH_2$-Trägerharz und —NH—Trägerharz bestehenden Gruppe ausgewählt ist.
8. Verfahren nach Anspruch 7, worin $X^1$ BOC, $X^2$ 2,6-Dichlorbenzyl, $X^3$ Bzl, $X^4$ Bzl, $X^5$ Bzl, $X^6$ Tos, $X^7$ 2-Chlorbenzyloxycarbonyl und $X^8$ —O—$CH_2$-Trägerharz ist.

9. Verfahren nach Anspruch 7, worin $X^1$ BOC, $X^2$ 2,6-Dichlorbenzyl, $X^3$ Bzl, $X^4$ Bzl, $X^5$ Bzl, $X^6$ Tos, $X^7$ 2-Chlorbenzyloxycarbonyl und $X^8$ —NH-Trägerharz ist.
10. Verfahren zur Erhöhung des Körpergewichtes über die Muskelmasse bei warmblütigen nicht menschlichen Tieren in der Landwirtschaft, wobei eine wirksame Menge einer Verbindung der Formel (I), nach Anspruch 1 zur Anregung der Freisetzung von STH zur Förderung des Wachstums des Tieres und nicht zu einem therapeutischen Zweck verabreicht wird.

11. Verfahren zur Verbesserung der Wassertierzucht durch Verabreichung einer Verbindung der Formel (I), nach Anspruch 1 an kaltblütige Tiere in einer zur Beschleunigung des Wachstums derselben wirksamen Menge und nicht zu einem therapeutischen Zweck.

**Revendications pour les Etats Contractants: BE, CH, DE, FR, GB, IT, LI, LU, NL, SE**

1. Un peptide synthétique ayant pour formule:

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-$R_{34}$-Asn-Gln-Glu-$R_{38}$-Gly-$R_{40}$-$R_{41}$-R

dans laquelle R est OH ou $NH_2$, $R_{34}$ est Ser ou Ala, $R_{38}$ est Arg ou Ser, $R_{40}$ est Ala ou Arg et $R_{41}$ est Arg, Arg-Ala, Arg-Ala-Arg, Arg-Ala-Arg-Leu ou dés-$R_{41}$, ou un fragment biologiquement actif de celui-ci, ou un sel non toxique de celui-ci.
2. Un peptide synthétique ayant la formule de la revendication 1 dans laquelle $R_{34}$ est Ser, $R_{38}$ est Arg et $R_{40}$ est Ala.
3. Un peptide synthétique ayant la formule de la revendication 1 dans laquelle $R_{34}$ est Ala, $R_{38}$ est Ser et $R_{40}$ est Arg.
4. Un peptide synthétique ayant la formule de l'une quelconque des revendications 1 à 3, dans laquelle $R_{41}$ est Arg-Ala-Arg-Leu.
5. Un peptide synthétique ayant la formule de l'une quelconque des revendications 1 à 4, dans laquelle R est $NH_2$.
6. Un peptide synthétique ayant la formule de l'une quelconque des revendications 1 à 4 dans laquelle R est OH.
7. Le peptide de la revendication 1 ayant pour formule

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-OH.

8. Un procédé pour améliorer en agriculture le poids corporel, exprimé par la masse musculaire, des animaux à sang chaud autres que l'homme, dans lequel une quantité efficace d'un composé comme défini dans l'une quelconque des revendications 1 à 7 est administrée pour stimuler la libération de la somatotrophine pour favoriser la croissance de l'animal et non à une fin thérapeutique quelconque.
9. Un procédé pour améliorer l'aquiculture par administration à des animaux à sang froid d'une quantité d'un composé comme défini dans l'une quelconque des revendications 1 à 7 efficace pour accélérer leur croissance et non à une fin thérapeutique quelconque.
10. Une composition pharmaceutique pour stimuler la libération de la somatotrophine chez un être humain comprenant un composé comme défini dans l'une quelconque des revendications 1 à 7 et un support liquide ou solide pharmaceutiquement acceptable de celui-ci.
11. Une composition vétérinaire pour stimuler la libération de la somatotrophine chez un animal autre

que l'homme comprenant un composé comme défini dans l'une quelconque des revendications 1 à 7 et un support liquide ou solide acceptable en médecine vétérinaire de celui-ci.

12. Un composé selon l'une quelconque des revendications 1 à 7 pour l'emploi dans la stimulation de la libération de la samatotrophine chez des êtres humains.

13. Un composé selon l'une quelconque des revendications 1 à 7 pour l'emploi dans l'accélération de la croissance des animaux à sang chaud autres que l'homme.

14. Un composé selon l'une quelconque des revendications 1 à 7 pour l'emploi en aquiculture pour accélérer la croissance des animaux à sang froid.

15. Un fragment biologiquement actif comme défini dans la revendication 1 caractérisé par la présence d'une séquence d'amino-acides:—

H-Tyr-Ala-Asp-Ala-Ile-Phe-Thr-Asn-Ser-Tyr-Arg-Lys-Val-Leu-Gly-
Gln-Leu-Ser-Ala-Arg-Lys-Leu-Leu-Gln-Asp-Ile-Met-.

16. Un peptide comme revendiqué dans la revendication 15 et ayant l'une des formules suivantes:—

(a) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-OH.
(b) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-Arg-Ala-Arg-Leu-OH.
(c) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-OH.
(d) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-OH.
(e) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-OH.
(f) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-OH.
(g) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-Arg-Ala-Arg-Leu-$NH_2$.
(h) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ala-Asn-Gln-Glu-Ser-Gly-Arg-OH.
(i) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-Arg-Gly-Ala-$NH_2$.
(j) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-OH.
(k) H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-Ser-Arg-Gln-Gln-Gly-Glu-Ser-Asn-Gln-Glu-$NH_2$.

17. Un sel non toxique d'un peptide comme revendiqué dans l'une quelconque des revendications 2 à 7, 15 et 16.

**Revendications pour l'Etat Contractant: AT**

1. Un procédé pour la préparation de composés définis par la formule (I):

H-Tyr-Ala-Asp-Ala-Ile-Phe-Thr-Asn-Ser-Tyr-Arg-Lys-Val-Leu-Gly-Gln-Leu-Ser-
Ala-Arg-Lys-Leu-Leu-Gln-Asp-Ile-Met-Ser-Arg-Gln-Gln-Gly-Glu-$R_{34}$-
Asn-Gln-Glu-$R_{38}$-Gly-$R_{40}$-$R_{41}$-R

ou leurs fragments biologiquement actifs, dans laquelle R est OH ou $NH_2$, $R_{34}$ est Ser ou Ala, $R_{38}$ est Arg ou

Ser, $R_{40}$ est Ala ou Arg et $R_{41}$ est Arg, Arg-Ala, Arg-Ala-Arg, Arg-Ala-Arg-Leu ou dés-$R_{41}$ comprenant (a) la formation d'un composé ayant au moins un groupe protecteur et la formule (II):

$X^1$-Tyr($X^2$)-Ala-Asp($X^3$)-Ala-Ile-Phe-Thr($X^4$)-Asn-Ser($X^5$)-Tyr($X^2$-Arg($X^6$)-Lys($X^7$)-Val-Leu-Gly-Gln-Leu-Ser($X^5$)-Ala-Arg($X^6$)-Lys($X^7$)-Leu-Leu-Gln-Asp($X^3$)-Ile-Met-Ser($X^5$)-Arg($X^6$)-Gln-Gln-Gly-Glu($X^3$)-$R_{34}$($X^5$)-Asn-Gln-Glu($X^3$)-$R_{38}$ ($X^5$ ou $X^6$)-Gly-$R_{40}$($X^6$)-$R_{41}$($X^6$)-$X^8$,

dans laquelle: ($X^1$), ($X^2$), ($X^3$), ($X^4$), ($X^5$), ($X^6$) et ($X^7$) sont chacun soit un hydrogène soit un groupe protecteur et ($X^8$) est soit un amide ou un hydroxyle soit un groupe protecteur, et (b) le clivage du ou des groupes protecteurs dudit composé de formule (II) pour fournir un peptide ayant la formule (I) ou un fragment biologiquement actif de celui-ci et, si on le désire, (c) la conversion du peptide obtenu en un de ses sels d'addition non toxiques.

2. Un procédé selon la revendication 1 dans lequel $R_{34}$ est Ser, $R_{38}$ est Arg et $R_{40}$ est Ala.

3. Un procédé selon l'une quelconque des revendications 1 ou 2, dans lequel $R_{41}$ est Arg-Ala-Arg-Leu.

4. Un procédé selon l'une quelconque des revendications 1 à 3 dans lequel R est $NH_2$.

5. Un procédé selon l'une quelconque des revendications 1 à 3 dans lequel R est OH.

6. Le procédé selon la revendication 1 dans lequel la formule du composé est

H-Tyr-Ala-Asp-Ala-Ile-Phe-Thr-Asn-Ser-Tyr-Arg-Lys-Val-Leu-Gly-Gln-Leu-Ser-Ala-Arg-Lys-Leu-Leu-Gln-Asp-Ile-Met-OH.

7. Un procédé selon l'une quelconque des revendications 1 à 6 dans lequel $X^1$ est un hydrogène ou un groupe α-amino-protecteur; $X^2$ est un hydrogène ou un groupe protecteur du groupe hydroxyle phénolique de Tyr; $X^3$ est un hydrogène ou un groupe protecteur du groupe carboxyle d'Asp ou Glu; $X^4$ est un hydrogène ou un groupe protecteur du groupe hydroxyle alcoolique de Thr; $X^5$ est un hydrogène ou un groupe protecteur du groupe hydroxyle alcoolique de Ser: $X^6$ est un hydrogène ou un groupe protecteur du groupe guanidino de Arg; $X^7$ est un hydrogène ou un groupe protecteur du groupe amino latéral de Lys; et $X^8$ est choisi dans la classe constituée par OH, $OCH_3$, les esters, les amides, les hydrazides, —O—$CH_2$—résine support et —NH-résine support.

8. Un procédé selon la revendication 7 dans lequel $X^1$ est BOC, $X^2$ est 2,6-dichlorobenzyle, $X^3$ est Bzl, $X^4$ est Bzl, $X^5$ est Bzl, $X^6$ est Tos, $X^7$ est 2-chlorobenzyloxycarbonyle et $X^8$ est O—$CH_2$-résine support.

9. Un procédé selon la revendication 7 dans lequel: $X^1$ est Boc, $X^2$ est 2,6-dichlorobenzyle, $X^3$ est Bzl, $X^4$ est Bzl, $X^5$ est Bzl, $X^6$ est Tos, $X^7$ est 2-chlorobenzyloxycarbonyle et $X^8$ est —NH-résine support.

10. Un procédé pour améliorer, en agriculture, le poids corporel, exprimé par la masse musculaire, des animaux à sang chaud autres que l'homme, dans lequel une quantité efficace d'un composé de formule (I) défini dans la revendication 1 est administrée pour stimuler la libération de la somatotrophine pour favoriser la croissance de l'animal, et non à une fin thérapeutique quelconque.

11. Un procédé pour améliorer l'aquiculture par administration à des animaux à sang froid d'une quantité d'un composé de formule (I) défini dans la revendication 1, efficace pour accélérer leur croissance et non à une fin thérapeutique quelconque.